Europäisches Patentamt

(19) European Patent Office

Office européen des brevets

(11) Publication number: **0 244 979**
**B1**

# (12) EUROPEAN PATENT SPECIFICATION

(45) Date of publication of the patent specification:
**19.09.90**

(21) Application number: **87303472.2**

(22) Date of filing: **21.04.87**

(51) Int. Cl.⁵: **A61F 11/08**

(54) Ear plugs.

(30) Priority: **29.04.86 GB 8610455**

(43) Date of publication of application:
**11.11.87 Bulletin 87/46**

(45) Publication of the grant of the patent:
**19.09.90 Bulletin 90/38**

(84) Designated Contracting States:
**BE CH DE FR GB IT LI NL**

(56) References cited:
**DE-A- 3 304 362**
**DE-C- 465 891**
**US-A- 2 573 923**
**US-A- 4 219 018**
**US-A- 4 314 553**
**US-A- 4 353 364**

(73) Proprietor: **SAFER SAFETY LIMITED, Unit 15 Dunston
Trading Estate Foxwood Road, Sheepbridge
Chesterfield S41 9RF(GB)**

(72) Inventor: **Salmon, John Douglas, 235 Springwood Lane,
High Green Sheffield S30 4JP(GB)**

(74) Representative: **Ford, Michael Frederick et al,
MEWBURN ELLIS & CO. 2/3 Cursitor Street, London
EC4A 1BQ(GB)**

## Description

The invention relates to ear plugs and in particular, though not exclusively, to ear plugs for workers in the food industry.

It is a particular problem that in many industries the continuous and loud noise of manufacturing equipment, and of packaging and conveying equipment, makes it necessary or advisable for ear plugs to be used, but that in the food processing industry the use of such safety devices presents a considerable danger of contamination to food if they fall into the foodstuffs being processed. This is because, although food processing production lines are invariably provided with detectors for detecting metallic foreign bodies, ear plugs are made of soft rubber or a synthetics plastics material and therefore cannot be detected by such safety systems.

US-A-2573923 discloses an ear plug that has an insert for stiffening purposes and it is stated that the insert may be made of a variety of materials, including metal. Such a plug would be detectable by metal-detecting systems, although it does not appear to have been adopted by the industry. The fact would remain, even if a lost plug could be detected, however, it is undesirable to allow it to come into contact with the foodstuffs.

It is known to provide a pair of ear plugs on the opposite ends of a length of cord so that if one ear plug becomes loose and falls from the ear of the person using it there is at least a good chance of it being retained on the end of the length of cord, but the known arrangements are not entirely satisfactory in this respect.

According to the present invention, there is provided a pair of ear plugs attached to the opposite ends of a length of cord and each plug containing within it a metallic part whereby if the plugs become misplaced during use in a food processing plant, they can be detected by metal detectors, the metallic part within each ear plug being constituted by a ferrule, the respective ferrules being secured on opposite ends of the length of cord by means of which the pair of plugs are connected together, the length of each said ferrule being less than the depth of the hole in the plug in which it is fitted, the plug being such that the outer part of the hole in the resilient plug is able to close slightly behind the ferrule to assist in retaining the respective ear plugs attached to the cord.

By the use of the invention, it is possible to produce a pair of ear plugs very economically which need make no compromise over their acoustic efficiency yet offer a high degree of security in that the individual plugs and their cord are locked together in a manner that makes accidental detachment extremely unlikely, while ensuring that they can be detected by conventional metal detection equipment if dropped by the wearer.

To combine that disclosure with DE-A-3304362 would simply result in the addition of a connecting cord to a pair of the earlier ear plugs. That is all that can be demonstrated to be obvious at the priority date of the present application.

One way of carrying out the invention is described in detail below with reference to drawings which illustrate, by way of example, one specific embodiment, in which:

Figure 1 is a view of a pair of ear plugs embodying the invention,

Figure 2 is an exploded view showing one of the plugs detached from the length of cord which normally connects them, and

Figure 3 is a sectional view, drawn to a somewhat enlarged scale, through one of the plugs when connected to the length of cord.

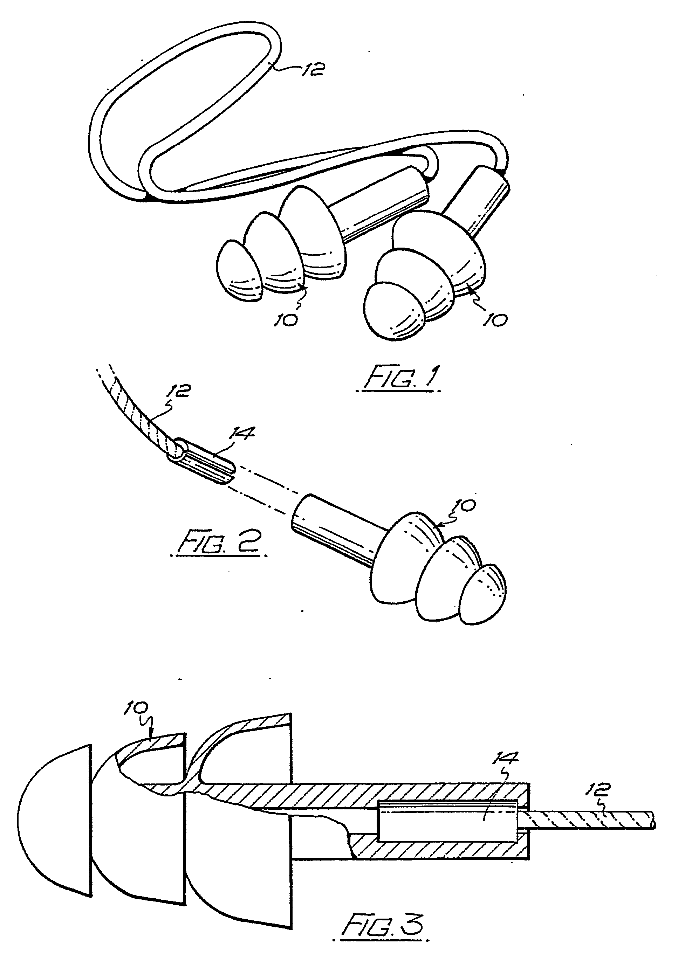

Referring now to the drawings, in Figure 1 there is illustrated a pair of ear plugs 10,10 connected together by a length of cord 12. The plugs are of conventional shape and have been moulded in a soft rubber or synthetic plastics material.

In Figure 2 there is illustrated how the opposite ends of the length of cord have each been provided with a metal ferrule 14. As shown in Figure 3, the length of each ferrule is less than the depth of the hole in the plug in which it is to be forced. Consequently, the ferrule is contained wholly within the plug and the outer part of the hole in the resilient plug has been able to close slightly to retain the ferrule within the plug. The arrangement is such that it is very unlikely that the plug could ever become detached by inadvertence from the length of cord. The metal ferrule, which is constituted by a roll of metal strip, is tightly crimped on the end of the cord in the way in which metal ferrules have sometimes been crimped on the ends of boot and shoe laces. Consequently, it is very unlikely that the length of cord will ever become detached from the metal ferrule held captive within the ear plug.

It has been found that if the pair of ear plugs just described should by inadvertence become misplaced during use in a food processing plant, or indeed if they are introduced mischievously into the foodstuffs, the metallic part will be detected by the usual metal detectors which are invariably used in such plant.

## Claims

A pair of ear plugs (10, 10) attached to the opposite ends of a length of cord (12) and each plug containing within it a metallic part whereby, if the plugs become misplaced during use in a food processing plant, they can be detected by metal detectors, characterised in that the metallic part within each ear plug is constituted by a ferrule (14), the respective ferrules being secured on opposite ends of the length of cord (12) by means of which the pair of plugs are connected together, the length of each said ferrule being less than the depth of the hole in the plug (10) in which it is fitted, the plug being moulded in a soft rubber or synthetic plastics material such that the outer part of the hole in the resilient plug is able to close slightly behind the ferrule to assist in retaining the respective ear plugs attached to the cord.

**Patentansprüche**

Gehörschutzstöpselpaar (10, 10), die an den gegenüberliegenden Enden einer Schnur (12) befestigt sind, und wobei jeder Stöpsel ein Metallteil enthält, wodurch die Stöpsel mit einem Metalldetektor geortet werden können, falls sie während der Verwendung in einem Lebensmittelbetrieb verlegt werden sollten, dadurch gekennzeichnet, daß das Metallteil in jedem Gehörschutzstöpsel von einer Klemmhülse (14) gebildet ist, wobei die betreffenden Klemmhülsen an gegenüberliegenden Enden der Schnur (12) befestigt sind, mit der das Gehörschutzstöpselpaar verbunden ist, wobei die Länge einer jeden Klemmhülse kleiner ist als die Tiefe der Bohrung in dem Stöpsel (10), in die sie eingesetzt ist, wobei der Stöpsel aus Weichgummi oder aus Kunststoff gegossen ist, so daß sich der äußere Teil der Bohrung des elastischen Stöpsels hinter der Klemmhülse leicht verjüngen kann, um dazu beizutragen, den betreffenden Gehörschutzstöpsel an der Schnur zu befestigen.

**Revendications**

Pair de tampons auriculaires (10, 10) reliés aux extrémités opposées d'un cordon (12) et chaque tampon contenant une partie métallique grâce à quoi, si les tampons s'égarent pendant l'utilisation dans une usine de traitement des aliments, ils peuvent être détectés par des détecteurs métalliques, caractérisée en ce que la partie métallique à l'intérieur de chaque tampon auriculaire est constituée par une virole (14), les viroles respectives étant fixées sur les extrémités opposées du cordon (12) par des moyens grâce à quoi les deux tampons sont reliés ensemble, la longueur de chaque dite virole étant inférieure à la profondeur du trou dans le tampon (10) dans lequel elle est logée, le tampon étant moulé dans un matériau plastique synthétique ou caoutchouc mou et tel que la partie extérieure du trou dans le tampon élastique puisse se fermer légèrement derrière la virole pour favoriser la retenue des tampons auriculaires respectifs en position attachés au cordon.

*FIG. 1*

*FIG. 2*

*FIG. 3*